# EUROPÄISCHE PATENTSCHRIFT

(11) **EP 1 633 745 B1**
(45) Veröffentlichungstag und Bekanntmachung des Hinweises auf die Patenterteilung: **02.07.2008**
(21) Anmeldenummer: 04732283.9
(22) Anmeldetag: 12.05.2004
(51) Int. Cl.: C07D 413/12, C07D 409/12, A61K 31/5377, A61P 7/02

(54) **AROYLSEMICARBAZIDDERIVATE GEGEN THROMBOEMBOLISCHE ERKRANKUNGEN**
AROYL SEMICARBAZIDE DERIVATIVES AGAINST THROMBOEMBOLIC DISEASES
DERIVES D'AROYL-SEMICARBAZIDE POUR LUTTER CONTRES LES MALADIES THROMBOEMBOLIQUES

(30) Priorität: 07.06.2003 DE 10325962
(43) Veröffentlichungstag der Anmeldung: 15.03.2006
(73) Patentinhaber: Merck Patent GmbH, 64293 Darmstadt (DE)
(72) Erfinder: MEDERSKI, Werner, 64673 Zwingenberg (DE); TSAKLAKIDIS, Christos, 69469 Weinheim (DE); DORSCH, Dieter, 64372 Ober-Ramstadt (DE); CEZANNE, Bertram, 64546 Mörfelden-Walldorf (DE); GLEITZ, Johannes, 64289 Darmstadt (DE)
(86) Internationale Anmeldenummer: PCT/EP2004/005088
(87) Internationale Veröffentlichungsnummer: WO 2004/108718

(56) Entgegenhaltungen:
- WO-A-02/06269
- DE-A- 10 040 783

## Beschreibung

Die Erfindung betrifft Verbindungen der Formel I 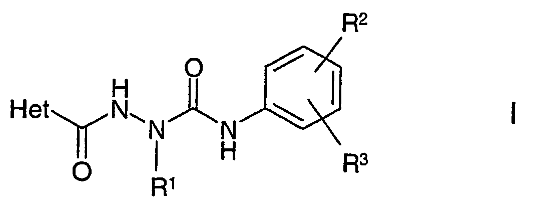 worin
- Het: einen ein- oder zweikernigen aromatischen Heterocyclus mit 1 bis 3 N-, O- und/oder S-Atomen, der ein- oder zweifach durch Hal substituiert ist,
- R¹: A, das ein-, zwei- oder dreimal durch S(O)ₘA, Ph, NH₂, NHA, NA₂, OH, OA, PO(OA)₂, Ethynyl, Vinyl oder O(CH₂)ₙPh substituiert sein kann,
- R²: H, Hal oder A,
- R³: 2-Oxo-piperidin-1-yl, 2-Oxo-pyrrolidin-1-yl, 2-Oxo-1*H*-pyridin-1-yl, 3-Oxo-morpholin-4-yl, 4-Oxo-*H*-pyridin-1-yl, 2-Oxo-1*H*-Pyrazin-1-yl, 2-Oxo-imidazolidin-1-yl, 2-Imino-piperidin-1-yl, 2-lmino-pyrrolidin-1-yl, 3-lmino-morpholin-4-yl, 2-Imino-imidazolidin-1-yl, 2-Imino-1 *H*-Pyrazin-1-yl, 2,6-Dioxo-piperidinl-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl,
- A: H, unverzweigtes, verzweigtes oder cyclisches Alkyl mit 1-10 C-Atomen,
- Ph: Phenyl,
- Hal: F, Cl, Br oder I,
- n: 1, 2, 3, 4, 5 oder 6,
- m: 0, 1 oder 2,
bedeuten,
sowie ihre pharmazeutisch verwendbaren Solvate, Salze und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I können weiterhin Inhibitoren der Gerinnungsfaktoren Faktor VIIa, Faktor IXa und Thrombin der Blutgerinnungskaskade sein.

Aromatische Amidinderivate mit antithrombotischer Wirkung sind z.B. aus der EP 0 540 051 B1, WO 98/28269, WO 00/71508, WO 00/71511, WO 00/71493, WO 00/71507, WO 00/71509, WO 00/71512, WO 00/71515 oder WO 00/71516 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt. Substituierte N-[(Aminoiminomethyl)-phenylalkyl]-azaheterocyclylamide als Faktor Xa Inhibitoren sind in WO 96/40679 beschrieben.

Regioisomere Verbindungen der erfindungsgemäßen Derivate sind in der DE 10040783.8 beschrieben (Verbindungen der Formel 7 in Syntheseschema 1).

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor VIIa, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.
Die Messung der Inhibierung von Thrombin kann z.B. nach der Methode von G. F. Cousins et al. in Circulation 1996, 94, 1705-1712 erfolgen.

Eine Inhibierung des Faktors Xa kann somit verhindern, daß Thrombin gebildet wird.
Die erfindungsgemäßen Verbindungen der Formel I sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Hauptmann et al. in Thrombosis and Haemostasis 1990, 63, 220-223 beschrieben.

Die Messung der Inhibierung von Faktor Xa kann z.B. nach der Methode von T. Hara et al. in Thromb. Haemostas. 1994, 71, 314-319 erfolgen.

Der Gerinnungsfaktor VIIa initiiert nach Bindung an Tissue Faktor den extrinsischen Teil der Gerinnungskaskade und trägt zur Aktivierung des Faktors X zu Faktor Xa bei. Eine Inhibierung von Faktor VIIa verhindert somit die Entstehung des Faktors Xa und damit eine nachfolgende Thrombinbildung.
Die Inhibierung des Faktors VIIa durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor Vlla wird z.B. von H. F. Ronning et al. in Thrombosis Research 1996, 84, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird.
Die Inhibierung von Faktor IXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. in Journal of Biological Chemistry 1998, 273, 12089-12094 beschrieben.

Die erfindungsgemäßen Verbindungen können weiterhin zur Behandlung von Tumoren, Tumorerkrankungen und/oder Tumormetastasen verwendet werden.
Ein Zusammenhang zwischen dem Tissuefaktor TF / Faktor VIIa und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt.
Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

Die Verbindungen der Formel I können als Arzneimittelwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale Ischämie, instabile Angina und auf Thrombose basierender Schlaganfall.
Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.
Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.

Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse.
Die Verbindungen finden ferner Verwendung bei der Reinigung von Kathetern und medizinischen Hilfsmitteln bei Patienten *in vivo,* oder als Antikoagulantien zur Konservierung von Blut, Plasma und anderen Blutprodukten *in vitro.* Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F.Morales-Asin et al., Headache, 40, 2000, 45-47).

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.
Besonders bevorzugt ist die gleichzeitige Gabe mit Aspirin, um ein Neuauftreten der Thrombenbildung zu verhindern.
Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Gegenstand der Erfindung sind die Verbindungen der Formel I und ihre Salze sowie ein Verfahren zur Herstellung von Verbindungen der Formel I nach den Ansprüchen 1-9 sowie ihrer pharmazeutisch verwendbaren Solvate, Salze und Stereoisomere, dadurch gekennzeichnet, daß man
a) eine Verbindung der Formel II 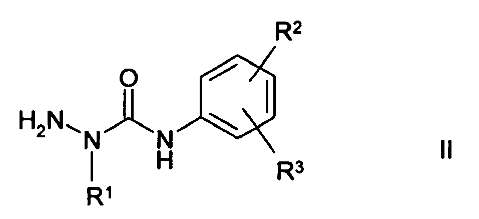
worin
R¹, R² und R³ die in Anspruch 1 angegebene Bedeutung haben,
mit einer Verbindung der Formel III

Het-CO-L III

worin
- L: Cl, Br, I oder eine freie oder reaktionsfähig funktionell abgewandelte OH-Gruppe bedeutet und
- Het: die in Anspruch 1 angegebene Bedeutung hat,
umsetzt,
und/oder
eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen ' Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, wie z.B. A, gilt, daß deren Bedeutungen unabhängig voneinander sind.
Vor- und nachstehend haben die Reste bzw. Parameter R, R¹, R², R³ die bei der Formel I angegebenen Bedeutungen, falls nicht ausdrücklich etwas anderes angegeben ist.

A bedeutet Alkyl, ist unverzweigt (linear), verzweigt oder cyclisch, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl oder Cyclopropylmethyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Cyclopentyl, Cyclohexyl, Cyclopropylmethyl, Trifluormethyl.
Cyclisches Alkyl bedeutet vorzugsweise Cyclopropyl, Cyclobutyl, Cylopentyl, Cyclohexyl oder Cycloheptyl.

Hal bedeutet vorzugsweise F, Cl oder Br, aber auch I.

Het bedeutet, unabhängig von der Substitution durch Hai, z.B. 2- oder 3-Furyl, 2- oder 3-Thienyl, 1-, 2- oder 3-Pyrrolyl, 1-, 2, 4- oder 5-lmidazolyl, 1-, 3-, 4- oder 5-Pyrazolyl, 2-, 4- oder 5-Oxazolyl, 3-, 4- oder 5-lsoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-lsoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-lsochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Het bedeutet besonders bevorzugt ein- oder zweifach durch Hal substituiertes Thienyl, Furyl, Pyrrolyl, Benzofuranyl, Benzo[b]thienyl, Thiazolyl oder Oxazolyl.

R¹ bedeutet vorzugsweise Alkyl mit 1, 2, 3, 4, 5 oder 6 C-Atomen, das durch S(O)ₘA, z.B. SO₂CH₃, durch NH₂, NHA, z.B. NHCH₃, durch NA₂, z.B. N(CH₃)₂, durch OH, OA, z.B. OCH₃, durch PO(OA)₂, z.B. PO(OCH₃)₂, durch Ethynyl, Phenyl, Vinyl oder O(CH₂)ₙPh substituiert sein kann.

R¹ bedeutet besonders bevorzugt Alkyl mit 1, 2, 3, 4, 5 oder 6 C-Atomen, das durch Ethynyl, Phenyl, OH oder OA substituiert sein kann.

R² bedeutet vorzugsweise z.B. H, Methyl oder F.

R³ bedeutet vorzugsweise 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1*H*-Pyrazin-1-yl, 2-Oxo-piperazin-1-yl oder 3-Oxo-2*H*-pyridazin-2-yl.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ie ausgedrückt werden, die der Formel I entsprechen und worin die nicht näher bezeichneten Reste die bei der Formel I angegebene Bedeutung haben, worin jedoch
- in la: R¹ Alkyl mit 1, 2, 3, 4, 5, oder 6 C-Atomen, das durch Ethynyl, Phenyl, OA, OH oder OA substituiert sein kann,
bedeutet;
- in Ib: R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1*H*-Pyrazin-1-yl, 2-Oxo-piperazin-1-yl oder 3-Oxo-2*H*-pyridazin-2-yl,
bedeutet;
- in Ic: R² H, Methyl oder F bedeutet;
- in Id: Het ein- oder zweifach durch Hal substituiertes Thienyl, Furyl, Pyrrolyl, Benzofuranyl, Benzo[b]thienyl, Thiazolyl oder Oxazolyl,
bedeutet;
- in le: Het ein- oder zweifach durch Hal substituiertes Thienyl, Furyl, Pyrrolyl, Benzofuranyl, Benzo[b]thienyl, Thiazolyl oder Oxazolyl,
R¹ Alkyl mit 1, 2, 3, 4, 5, oder 6 C-Atomen, das durch Ethynyl, Phenyl, OA, OH oder OA substituiert sein kann,
R² H, Hal oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1*H*-Pyrazin-1-yl, 2-Oxo-piperazin-1-yl oder 3-Oxo-2*H*-pyridazin-2-yl,
A H, unverzweigtes, verzweigtes oder cyclisches Alkyl mit 1-10 C-Atomen,
Ph Phenyl,
Hal F, Cl, Br oder I,
n 1, 2, 3, 4, 5 oder 6,
m 0, 1 oder 2,
bedeuten;
sowie ihre pharmazeutisch verwendbaren Solvate, Salze und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel umsetzt.

Verbindungen der Formel I können vorzugsweise erhalten werden, indem man Verbindungen der Formel II mit Verbindungen der Formel III umsetzt. Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Wasser; Kohlenwasserstoffe wie Hexan, Petrolether, Benzol, Toluol oder Xylol; chlorierte Kohlenwasser, Salze stoffe wie Trichlorethylen, 1,2-Dichlorethan, Tetrachlorkohlenstoff, Chloroform oder Dichlormethan; Alkohole wie Methanol, Ethanol, Iso, Salze propanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Ausgangsverbindungen der Formeln II und III sind in der Regel bekannt. Sind sie neu, so können sie aber nach an sich bekannten Methoden hergestellt werden.

In den Verbindungen der Formel III bedeutet L vorzugsweise Cl, Br, I oder eine reaktionsfähig abgewandelte OH-Gruppe wie z:B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Eine Base der Formel 1 kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Diese Zubereitungen können in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens, Migräne, Tumoren, Tumorerkrankungen und/oder Tumormetastasen verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Solvate, Salze und Stereoisomere, einschließlich deren Mischungen in allen Verhältnissen,
   und
(b) einer wirksamen Menge eines weiteren Arzneimittels.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Solvate, Salze und Stereoisomere, einschließlich deren Mischungen in allen Verhältnissen,
und einer wirksamen Menge eines weiteren Arzneimittels gelöst oder in lyophylisierter Form vorliegt.

Gegenstand der Erfindung ist ferner die Verwendung von Verbindungen der Formel I und/oder ihrer pharmazeutisch verwendbaren Solvate, Salze und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen, in Kombination mit mindestens einem weiteren Arzneimittelwirkstoff.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: EI (Elektronenstoß-Ionisation) M⁺
FAB (Fast Atom Bombardment) (M+H)⁺
ESI (Electrospray lonization) (M+H)⁺ (wenn
nichts anderes angegeben)

### Beispiel 1

Die Herstellung von 1-(5-Chlor-thien-2-ylcarbonyl)-4-[4-(3-oxo-morpholin-4-yl)-phenyl]-2-propyl-semicarbazid erfolgt analog nachstehendem Schema: 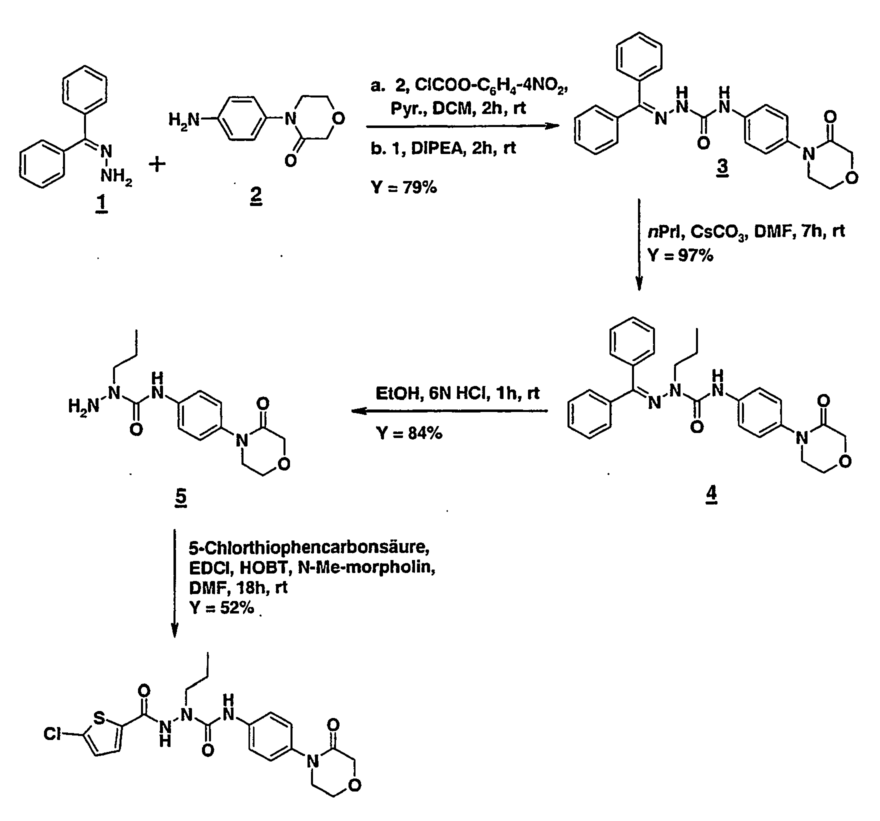
1. Eine Mischung aus 2,0 g (10,4 mmol) 4-(4-Amino-phenyl)-morpholin-3-on **2**, 2,1 g (10,4 mmol) 4-Nitro-phenyl-chloroformiat, 0,84 mL (10,4 mmol) Pyridin in 50 mL Dichlormethan wird 1 Stunde bei Raumtemperatur gerührt. Diese Mischung wird nacheinander mit 2,04 g (10,4 mmol) Benzophenonhydrazon **1** und 5,3 mL (31,2 mmol) Ethyldiisopropylamin versetzt. Die Suspension wird 2 Stunden bei Raumtemperatur gerührt, mit Ethylacetat verdünnt und mit 0,5 N Natriumhydroxidlösung gewaschen. Danach wird wie üblich aufgearbeitet und man erhält so 3,94 g (79%) 1-Diphenylmethylen-4-[4-(3-oxo-4-morpholinyl)-phenyl]-semicarbazid 3 als farblose Kristalle mit einem Schmelzpunkt von 241-243°; MS-El (M⁺) = 414 (8%), 165 (100%).
2. Eine Suspension von 1,0 g (2,4 mmol) Semicarbazid **3** und 0,98 g ( 3,0 mmol) Cäsiumcarbonat in 30 mL Acetonitril wird 0,5 Stunden bei Raumtemperatur gerührt. Diese Mischung wird versetzt mit 0,29 mL (3,0 mmol) 1-lodpropan und für weitere 7 Stunden gerührt. Nach Versetzen mit 150 mL eiskaltem Wasser setzt sich ein Niederschlag ab, der wie üblich aufgearbeitet wird. So erhält man 1,07 g (97%) 1-Diphenylmethylen-4-[4-(3-oxo-4-morpholinyl)-phenyl]-2-propylsemicarbazid **4** als farblose Kristalle mit einem Schmelzpunkt von 75-77°; MS-EI (M⁺) = 456 (2%), 180 (100%).
3. Eine Lösung von 0,5 g (1,1 mmol) Propylsemicarbazid **4** in 20 mL Ethanol wird mit 5,0 mL 37%ige Hydrogenchloridlösung versetzt und 1 Stunde bei Raumtemperatur gerührt. Nach Verdünnen der Reaktionslösung mit 30 mL Wasser wird wie üblich aufgearbeitet. So erhält man 0,27 g (84%) 4-[4-(3-Oxo-4-morpholinyl)-phenyl]-2-propylsemicarbazid **5** als farblose Kristalle mit einem Schmelzpunkt von 127-129°; MS-EI (M⁺) = 318 (6%), 218 (100%).
4. 100,0 mg (0,342 mmol) Semicarbazid **5**, 55,6 mg (0,342 mmol) 5-Chlorthiophen-2-carbonsäure, 66,2 mg (0,342 mmol) N-(3-Dimethylaminopropyl)-N'-ethylcarbodiimidhydrochlorid und 36,2 mg (0,342 mmol) 1-Hydroxybenzotriazol werden in 10 mL DMF gelöst und bei RT mit 38,0 µL (0,342 mmol) 4-Methylmorpholin versetzt. Nach 18 h rühren bei RT wird auf 75 mL Eiswasser gegossen und wie üblich aufgearbeitet. So erhält man 77,0 mg (51,5%) 1-(5-Chlor-thien-2-ylcarbonyl)-4-[4-(3-oxo-morpholin-4-yl)-phenyl]-2-propyl-semicarbazid ("A1") als farblose Kristalle mit einem Schmelzpunkt von 101-102°; MS-ESI (M+H⁺) = 437.

Analog erhält man die nachstehenden Verbindungen
1-(5-Chlor-thien-2-ylcarbonyl)-4-[3-methyl-4-(3-oxo-morpholin-4-yl)-phenyl]-2-(prop-2-ynyl)-semicarbazid ("A2"),
1-(3-Chlor-thien-2-ylcarbonyl)-4-[3-methyl-4-(3-oxo-morpholin-4-yl)-phenyl]-2-benzyl-semicarbazid ("A3"),
1-(5-Brom-thien-2-yicarbonyl)-4-[3-methyl-4-(3-oxo-morpholin-4-yl)-phenyl]-2-benzyl-semicarbazid,
1-(3-Chlor-benzo[b]thienyl-2-ylcarbonyl)-4-[3-methyl-4-(3-oxo-morpholin-4-yl)-phenyl]-2-benzyl-semicarbazid,
1-(5-Chlor-thien-2-ylcarbonyl)-4-[3-methyl-4-(3-oxo-morpholin-4-yl)-phenyl]-2-benzyl-semicarbazid,
1-(5-Brom-thien-2-ylcarbonyl)-4-[3-fluor-4-(3-oxo-morpholin-4-yl)-phenyl]-2-(2-methoxy-ethyl)-semicarbazid,
1-(3-Chlor-benzo[b]thienyl-2-ylcarbonyl)-4-[3-fluor-4-(3-oxo-morpholin-4-yl)-phenyl]-2-(2-methoxy-ethyl)-semicarbazid,
1-(3-Chlor-thien-2-ylcarbonyl)-4-[3-fluor-4-(3-oxo-morpholin-4-yl)-phenyl]-2-(2-methoxy-ethyl)-semicarbazid,
1-(5-Chlor-thien-2-ylcarbonyl)-4-[3-fluor-4-(3-oxo-morpholin-4-yl)-phenyl]-2-(2-methoxy-ethyl)-semicarbazid,
1-(3-Chlor-thien-2-ylcarbonyl)-4-[4-(2-oxo-1*H*-pyrazin-1-yl)-phenyl]-2-cyclopropylmethyl-semicarbazid,
1-(3-Chlor-thien-2-ylcarbonyl)-4-[4-(2-oxo-1*H*-pyridin-1-yl)-phenyl]-2-cyclopropylmethyl-semicarbazid,
1-(3-Chlor-thien-2-ylcarbonyl)-4-[3-methyl-4-(3-oxo-morpholin-4-yl)-phenyl]-2-cyclopropylmethyl-semicarbazid,
1-(5-Chlor-thien-2-ylcarbonyl)-4-[3-methyl-4-(3-oxo-morpholin-4-yl)-phenyl]-2-(2-methoxy-ethyl)-semicarbazid,
1-(5-Chlor-thien-2-ylcarbonyl)-4-[3-methyl-4-(3-oxo-morpholin-4-yl)-phenyl]-2-cyclopropylmethyl-semicarbazid,
1-(5-Brom-thien-2-ylcarbonyl)-4-[3-methyl-4-(3-oxo-morpholin-4-yl)-phenyl]-2-cyclopropylmethyl-semicarbazid.

### Pharmakologische Daten

Affinität zu Rezeptoren

**Tabelle 1**

| Verbindung Nr. | FXa-IC₅₀[nM] | TF/FVlla-IC₅₀ [nM] |
|---|---|---|
| "A1" | 390.0 | 830.0 |
| "A2" | 570.0 | 1000.0 |
| "A3" | 87.0 | 180.0 |
| | | |
| | | |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄· 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 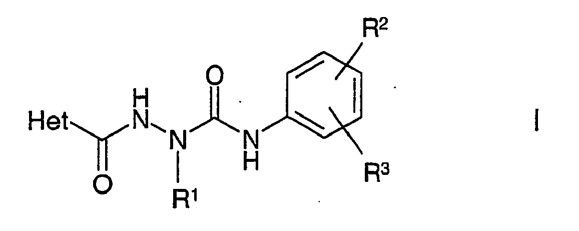 worin
Het einen ein- oder zweikernigen aromatischen Heterocyclus mit 1 bis 3 N-, O- und/oder S-Atomen, der ein- oder zweifach durch Hal substituiert ist,
R¹ A, das ein-, zwei- oder dreimal durch S(O)ₘA, Ph, NH₂, NHA, NA₂, OH, OA, PO(OA)₂, Ethynyl, Vinyl oder O(CH₂)ₙPh substituiert sein kann,
R² H, Hal oder A,
R³ 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2-Oxo-1*H-*Pyrazin-1-yl, 2-Oxo-imidazolidin-1-yl, 2-Imino-piperidin-1-yl, 2-Imino-pyrrolidin-1-yl, 3-lmino-morpholin-4-yl, 2-lmino-imidazolidin-1-yl, 2-Imino-1*H*-Pyrazin-1-yl, 2,6-Dioxo-piperidinl-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl,
A H, unverzweigtes, verzweigtes oder cyclisches Alkyl mit 1-10 C-Atomen,
Ph Phenyl,
Hal F, Cl, Br oder I,
n 1, 2, 3, 4, 5 oder 6,
m 0, 1 oder 2,
bedeuten,
sowie ihre pharmazeutisch verwendbaren Solvate, Salze und Stereoisomere, einschließlich deren Mischungen in allen Verhältnissen.

2. Verbindungen nach Anspruch 1, worin
R¹ Alkyl mit 1, 2, 3, 4, 5, oder 6 C-Atomen, das durch Ethynyl, Phenyl, OA, OH oder OA substituiert sein kann,
bedeutet,
sowie ihre pharmazeutisch verwendbaren Solvate, Salze und Stereoisomere, einschließlich deren Mischungen in allen Verhältnissen.

3. Verbindungen nach Anspruch 1, worin
R³ 2-Oxo-piperidin-1-yl, 2-Oxo-pyrrolidin-1-yl, 2-Oxo-1*H-*pyridin-1-yl 3-Oxo-morpholin-4-yl, 4-Oxo-1*H*-pyridin-1-yl, 2-Oxo-1*H-*Pyrazin-1-yl, 2-Oxo-imidazolidin-1-yl, 2-Imino-piperidin-1-yl, 2-lmino-pyrrolidin-1-yl, 3-Imino-morpholin-4-yl, 2-Imino-imidazolidin-1-yl, 2-Imino-1*H*-Pyrazin-1-yl, 2-Oxo-piperazin-1-yl oder 3-Oxo-2*H*-pyridazin-2-yl,
bedeutet,
sowie ihre pharmazeutisch verwendbaren Solvate, Salze und Stereoisomere, einschließlich deren Mischungen in allen Verhältnissen.

4. Verbindungen gemäß einem oder mehreren der Ansprüche 1-3, worin
R² H, Methyl oder F bedeutet,
sowie ihre pharmazeutisch verwendbaren Solvate, Salze und Stereoisomere, einschließlich deren Mischungen in allen Verhältnissen.

5. Verbindungen gemäß einem oder mehreren der Ansprüche 1-4, worin
Het ein- oder zweifach durch Hal substituiertes Thienyl, Furyl, Pyrrolyl, Benzofuranyl, Benzo[b]thienyl, Thiazolyl oder Oxazolyl,
bedeutet,
sowie ihre pharmazeutisch verwendbaren Solvate, Salze und Stereoisomere, einschließlich deren Mischungen in allen Verhältnissen.

6. Verbindungen gemäß einem oder mehreren der Ansprüche 1-5, worin
Het ein- oder zweifach durch Hal substituiertes Thienyl, Furyl, Pyrrolyl, Benzofuranyl, Benzo[b]thienyl, Thiazolyl oder Oxazolyl,
R¹ Alkyl mit 1, 2, 3, 4, 5, oder 6 C-Atomen, das durch Ethynyl, Phenyl, OA, OH oder OA substituiert sein kann,
R² H, Hal oder A,
R³ 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2-Oxo-1*H-*Pyrazin-1-yl, 2-Oxo-imidazolidin-1-yl, 2-Imino-piperidin-1-yl, 2-lmino-pyrrolidin-1-yl, 3-lmino-morpholin-4-yl, 2-lmino-imidazolidin-1-yl, 2-Imino-1*H*-Pyrazin-1-yl, 2-Oxo-piperazin-1-yl oder 3-Oxo-2*H*-pyridazin-2-yl,
A H, unverzweigtes, verzweigtes oder cyclisches Alkyl mit 1-10 C-Atomen,
Ph Phenyl,
Hal F, Cl, Br oder I,
n 1, 2, 3, 4, 5 oder 6,
m 0, 1 oder 2,
bedeuten,
sowie ihre pharmazeutisch verwendbaren Solvate, Salze und Stereoisomere, einschließlich deren Mischungen in allen Verhältnissen.

7. Verbindungen gemäß Anspruch 1 ausgewählt aus der Gruppe
1-(5-Chlor-thien-2-ylcarbonyl)-4-[4-(3-oxo-morpholin-4-yl)-phenyl]-2-propyl-semicarbazid,
1-(5-Chlor-thien-2-ylcarbonyl)-4-[3-methyl-4-(3-oxo-morpholin-4-yl)-phenyl]-2-(prop-2-ynyl)-semicarbazid,
1-(3-Chlor-thien-2-ylcarbonyl)-4-[3-methyl-4-(3-oxo-morpholin-4-yl)-phenyl]-2-benzyl-semicarbazid,
1-(5-Brom-thien-2-ylcarbonyl)-4-[3-methyl-4-(3-oxo-morpholin-4-yl)-phenyl]-2-benzyl-semicarbazid,
1-(3-Chlor-benzo[b]thienyl-2-ylcarbonyl)-4-[3-methyl-4-(3-oxo-morpholin-4-yl)-phenyl]-2-benzyl-semicarbazid,
1-(5-Chlor-thien-2-ylcarbonyl)-4-[3-methyl-4-(3-oxo-morpholin-4-yl)-phenyl]-2-benzyl-semicarbazid,
1-(5-Brom-thien-2-ylcarbonyl)-4-[3-fluor-4-(3-oxo-morpholin-4-yl)-phenyl]-2-(2-methoxy-ethyl)-semicarbazid,
1-(3-Chlor-benzo[b]thienyl-2-ylcarbonyl)-4-[3-fluor-4-(3-oxo-morpholin-4-yl)-phenyl]-2-(2-methoxy-ethyl)-semicarbazid,
1-(3-Chlor-thien-2-ylcarbonyl)-4-[3-fluor-4-(3-oxo-morpholin-4-yl)-phenyl]-2-(2-methoxy-ethyl)-semicarbazid,
1-(5-Chlor-thien-2-ylcarbonyl)-4-[3-fluor-4-(3-oxo-morpholin-4-yl)-phenyl]-2-(2-methoxy-ethyl)-semicarbazid,
1-(3-Chlor-thien-2-ylcarbonyl)-4-[4-(2-oxo-1*H*-pyrazin-1-yl)-phenyl]-2-cyclopropylmethyl-semicarbazid,
1-(3-Chlor-thien-2-ylcarbonyl)-4-[4-(2-oxo-1*H*-pyridin-1-yl)-phenyl]-2-cyclopropylmethyl-semicarbazid,
1-(3-Chlor-thien-2-ylcarbonyl)-4-[3-methyl-4-(3-oxo-morpholin-4-yl)-phenyl]-2-cyclopropylmethyl-semicarbazid,
1-(5-Chlor-thien-2-ylcarbonyl)-4-[3-methyl-4-(3-oxo-morpholin-4-yl)-phenyl]-2-(2-methoxy-ethyl)-semicarbazid,
1-(5-Chlor-thien-2-ylcarbonyl)-4-[3-methyl-4-(3-oxo-morpholin-4-yl)-phenyl]-2-cyclopropylmethyl-semicarbazid,
1-(5-Brom-thien-2-ylcarbonyl)-4-[3-methyl-4-(3-oxo-morpholin-4-yl)-phenyl]-2-cyclopropylmethyl-semicarbazid,
sowie ihre pharmazeutisch verwendbaren Solvate, Salze und Stereoisomere, einschließlich deren Mischungen in allen Verhältnissen.

8. Verfahren zur Herstellung von Verbindungen der Formel I nach den Ansprüchen 1-7 sowie ihrer pharmazeutisch verwendbaren Solvate, Salze und Stereoisomere, **dadurch gekennzeichnet, daß** man
a) eine Verbindung der Formel II 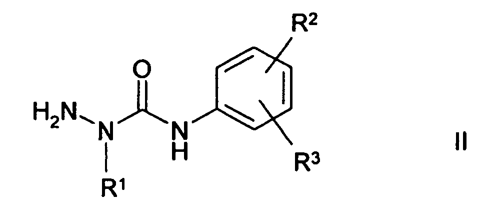 worin
R¹, R² und R³ die in Anspruch 1 angegebene Bedeutung haben,
mit einer Verbindung der Formel III
Het-CO-L III
worin
L Cl, Br, I oder eine freie oder reaktionsfähig funktionell abgewandelte OH-Gruppe bedeutet und
Het die in Anspruch 1 angegebene Bedeutung hat,
umsetzt,
und/oder
eine Base oder Säure der Formel I in eines ihrer Salze umwandelt.

9. Verbindungen der Formel I nach einem oder mehreren der Ansprüche 1 bis 7 als Inhibitoren des Koagulationsfaktors Xa.

10. Verbindungen der Formel I nach einem oder mehreren der Ansprüche 1 bis 7 als Inhibitoren des Koagulationsfaktors VIIa.

11. Arzneimittel, enthaltend mindestens eine Verbindung der Formel I nach einem oder mehreren der Ansprüche 1 bis 7 und/oder ihre pharmazeutisch verwendbaren Solvate, Salze und Stereoisomere, einschließlich deren Mischungen in allen Verhältnissen, sowie gegebenenfalls Träger- und/oder Hilfsstoffe.

12. Arzneimittel enthaltend mindestens eine Verbindung der Formel I gemäß einem oder mehreren der Ansprüche 1 bis 7 und/oder ihre pharmazeutisch verwendbaren Solvate, Salze und Stereoisomere, einschließlich deren Mischungen in allen Verhältnissen, und mindestens einen weiteren Arzneimittelwirkstoff.

13. Verwendung von Verbindungen gemäß der Ansprüche 1 bis 7 und/oder ihre physiologisch unbedenklichen Salze, Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen.

14. Set (Kit), bestehend aus getrennten Packungen von
(a) einer wirksamen Menge an einer Verbindung der Formel I gemäß einem oder mehreren der Ansprüche 1 bis 7 und/oder ihrer pharmazeutisch verwendbaren Solvate, Salze und Stereoisomere, einschließlich deren Mischungen in allen Verhältnissen,
und
(b) einer wirksamen Menge eines weiteren Arzneimittelswirkstoffs.

15. Verwendung von Verbindungen der Formel I gemäß einem oder mehreren der Ansprüche 1 bis 7 und/oder ihrer pharmazeutisch verwendbaren Solvate, Salze und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen,
in Kombination mit mindestens einem weiteren Arzneimittelwirkstoff.

## Claims

1. Compounds of the formula I 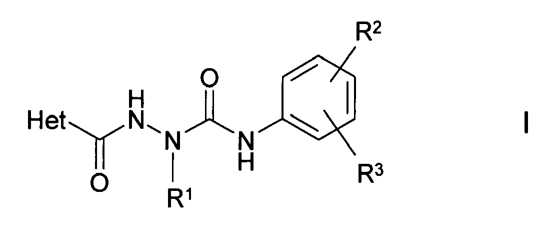 in which
Het denotes a mono- or bicyclic aromatic heterocycle having 1 to 3 N, O and/or S atoms which is mono- or disubstituted by Hal,
R¹ denotes A, which may be mono-, di- or trisubstituted by S(O)ₘA, Ph, NH₂, NHA, NA₂, OH, OA, PO(OA)₂, ethynyl, vinyl or O(CH₂)ₙPh,
R² denotes H, Hal or A,
R³ denotes 2-oxopiperidin-1-yl, 2-oxopyrrolidin-1-yl, 2-oxo-1*H-*pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2-iminopiperidin-1-yl, 2-iminopyrrolidin-1-yl, 3-iminomorpholin-4-yl, 2-imino-imidazolidin-1-yl, 2-imino-1*H*-pyrazin-1-yl, 2,6-dioxopiperidin-1-yl, 2-oxopiperazin-1-yl, 2,6-dioxopiperazin-1-yl, 2,5-dioxo-pyrrolidin-1-yl, 2-oxo-1,3-oxazolidin-3-yl, 3-oxo-2*H*-pyridazin-2-yl, 2-caprolactam-1-yl (= 2-oxoazepan-1-yl), 2-azabicyclo-[2.2.2]octan-3-on-2-yl, 5,6-dihydro-1*H*-pyrimidin-2-oxo-1-yl, 2-oxo-1,3-oxazinan-3-yl or 4*H*-1,4-oxazin-4-yl,
A denotes H, unbranched, branched or cyclic alkyl having 1-10 C atoms,
Ph denotes phenyl,
Hal denotes F, Cl, Br or I,
n denotes 1, 2, 3, 4, 5 or 6,
m denotes 0, 1 or 2,
and pharmaceutically usable solvates, salts and stereoisomers thereof, including mixtures thereof in all ratios.

2. Compounds according to Claim 1, in which
R¹ denotes alkyl having 1, 2, 3, 4, 5 or 6 C atoms, which may be substituted by ethynyl, phenyl, OA, OH or OA,
and pharmaceutically usable solvates, salts and stereoisomers thereof, including mixtures thereof in all ratios.

3. Compounds according to Claim 1 in which
R³ denotes 2-oxopiperidin-1-yl, 2-oxopyrrolidin-1-yl, 2-oxo-1*H-*pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2-iminopiperidin-1-yl, 2-iminopyrrolidin-1-yl, 3-iminomorpholin-4-yl, 2-imino-imidazolidin-1-yl, 2-imino-1*H*-pyrazin-1-yl, 2-oxopiperazin-1-yl or 3-oxo-2H-pyridazin-2-yl,
and pharmaceutically usable solvates, salts and stereoisomers thereof, including mixtures thereof in all ratios.

4. Compounds according to one or more of Claims 1-3 in which
R² denotes H, methyl or F,
and pharmaceutically usable solvates, salts and stereoisomers thereof, including mixtures thereof in all ratios.

5. Compounds according to one or more of Claims 1-4 in which
Het denotes thienyl, furyl, pyrrolyl, benzofuranyl, benzo[b]thienyl, thiazolyl or oxazolyl, each of which is mono- or disubstituted by Hal,
and pharmaceutically usable solvates, salts and stereoisomers thereof, including mixtures thereof in all ratios.

6. Compounds according to one or more of Claims 1-5 in which
Het denotes thienyl, furyl, pyrrolyl, benzofuranyl, benzo[b]thienyl, thiazolyl or oxazolyl, each of which is mono- or disubstituted by Hal,
R¹ denotes alkyl having 1, 2, 3, 4, 5 or 6 C atoms, which may be substituted by ethynyl, phenyl, OA, OH or OA,
R² denotes H, Hal or A,
R³ denotes 2-oxopiperidin-1-yl, 2-oxopyrrolidin-1-yl, 2-oxo-1*H-*pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2-iminopiperidin-1-yl, 2-iminopyrrolidin-1-yl, 3-iminomorpholin-4-yl, 2-imino-imidazolidin-1-yl, 2-imino-1*H*-pyrazin-1-yl, 2-oxopiperazin-1-yl or 3-oxo-2*H*-pyridazin-2-yl,
A denotes H, unbranched, branched or cyclic alkyl having 1-10 C atoms,
Ph denotes phenyl,
Hal denotes F, Cl, Br or I,
n denotes 1, 2, 3, 4, 5 or 6,
m denotes 0, 1 or 2,
and pharmaceutically usable solvates, salts and stereoisomers thereof, including mixtures thereof in all ratios.

7. Compounds according to Claim 1 selected from the group
1-(5-chlorothien-2-ylcarbonyl)-4-[4-(3-oxomorpholin-4-yl)phenyl]-2-propylsemicarbazide,
1-(5-chlorothien-2-ylcarbonyl)-4-[3-methyl-4-(3-oxomorpholin-4-yl)-phenyl]-2-(prop-2-ynyl)semicarbazide,
1-(3-chlorothien-2-ylcarbonyl)-4-[3-methyl-4-(3-oxomorpholin-4-yl)-phenyl]-2-benzylsemicarbazide,
1-(5-bromothien-2-ylcarbonyl)-4-[3-methyl-4-(3-oxomorpholin-4-yl)-phenyl]-2-benzylsemicarbazide,
1-(3-chlorobenzo[b]thienyl-2-ylcarbonyl)-4-[3-methyl-4-(3-oxomorpholin-4-yl)phenyl]-2-benzylsemicarbazide,
1-(5-chlorothien-2-ylcarbonyl)-4-[3-methyl-4-(3-oxomorpholin-4-yl)-phenyl]-2-benzylsemicarbazide,
1-(5-bromothien-2-ylcarbonyl)-4-[3-fluoro-4-(3-oxomorpholin-4-yl)-phenyl]-2-(2-methoxyethyl)semicarbazide,
1-(3-chlorobenzo[b]thienyl-2-ylcarbonyl)-4-[3-fluoro-4-(3-oxomorpholin-4-yl)phenyl]-2-(2-methoxyethyl)semicarbazide,
1-(3-chlorothien-2-ylcarbonyl)-4-[3-fluoro-4-(3-oxomorpholin-4-yl)-phenyl]-2-(2-methoxyethyl)semicarbazide,
1-(5-chlorothien-2-ylcarbonyl)-4-[3-fluoro-4-(3-oxomorpholin-4-yl)-phenyl]-2-(2-methoxyethyl)semicarbazide,
1-(3-chlorothien-2-ylcarbonyl)-4-[4-(2-oxo-1*H*-pyrazin-1-yl)phenyl]-2-cyclopropylmethylsemicarbazide,
1-(3-chlorothien-2-ylcarbonyl)-4-[4-(2-oxo-1*H*-pyridin-1-yl)phenyl]-2-cyclopropylmethylsemicarbazide,
1-(3-chlorothien-2-ylcarbonyl)-4-[3-methyl-4-(3-oxomorpholin-4-yl)-phenyl]-2-cyclopropylmethylsemicarbazide,
1-(5-chlorothien-2-ylcarbonyl)-4-[3-methyl-4-(3-oxomorpholin-4-yl)-phenyl]-2-(2-methoxyethyl)semicarbazide,
1-(5-chlorothien-2-ylcarbonyl)-4-[3-methyl-4-(3-oxomorpholin-4-yl)-phenyl]-2-cyclopropylmethylsemicarbazide,
1-(5-bromothien-2-ylcarbonyl)-4-[3-methyl-4-(3-oxomorpholin-4-yl)-phenyl]-2-cyclopropylmethylsemicarbazide,
and pharmaceutically usable solvates, salts and stereoisomers thereof, including mixtures thereof in all ratios.

8. Process for the preparation of compounds of the formula I according to Claims 1-7 and pharmaceutically usable solvates, salts and stereoisomers thereof, **characterised in that**
a) a compound of the formula II 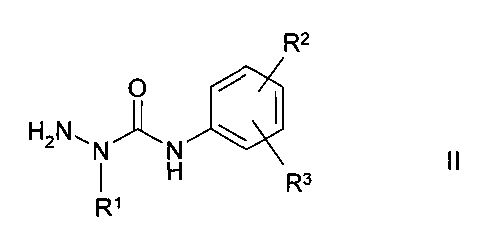
in which
R¹, R² and R³ have the meaning indicated in Claim 1,
is reacted with a compound of the formula III
Het-CO-L III
in which
L denotes Cl, Br, I or a free or reactively functionally modified OH group, and
Het has the meaning indicated in Claim 1,
and/or
a base or acid of the formula I is converted into one of its salts.

9. Compounds of the formula I according to one or more of Claims 1 to 7 as inhibitors of coagulation factor Xa.

10. Compounds of the formula I according to one or more of Claims 1 to 7 as inhibitors of coagulation factor VIIa.

11. Medicaments comprising at least one compound of the formula I according to one or more of Claims 1 to 7 and/or pharmaceutically usable solvates, salts and stereoisomers thereof, including mixtures thereof in all ratios, and, if desired, excipients and/or adjuvants.

12. Medicaments comprising at least one compound of the formula I according to one or more of Claims 1 to 7 and/or pharmaceutically usable solvates, salts and stereoisomers thereof, including mixtures thereof in all ratios, and at least one further medicament active ingredient.

13. Use of compounds according to Claims 1 to 7 and/or physiologically acceptable salts and solvates thereof for the preparation of a medicament for the treatment of thromboses, myocardial infarction, arteriosclerosis, inflammations, apoplexy, angina pectoris, restenosis after angioplasty, claudicatio intermittens, migraine, tumours, tumour diseases and/or tumour metastases.

14. Set (kit) consisting of separate packs of
(a) an effective amount of a compound of the formula I according to one or more of Claims 1 to 7 and/or pharmaceutically usable solvates, salts and stereoisomers thereof, including mixtures thereof in all ratios,
and
(b) an effective amount of a further medicament active ingredient.

15. Use of compounds of the formula I according to one or more of Claims 1 to 7 and/or pharmaceutically usable solvates, salts and stereoisomers thereof, including mixtures thereof in all ratios, for the preparation of a medicament for the treatment of thromboses, myocardial infarction, arteriosclerosis, inflammations, apoplexy, angina pectoris, restenosis after angioplasty, claudicatio intermittens, migraine, tumours, tumour diseases and/or tumour metastases, in combination with at least one further medicament active ingredient.

## Revendications

1. Composés de formule I 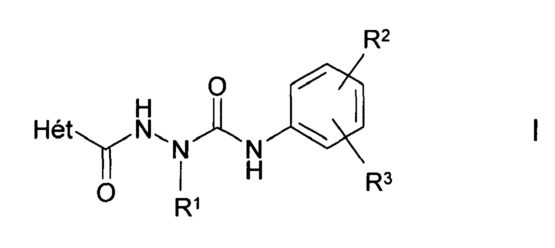 dans laquelle
Hét désigne un hétérocycle mono- ou bicyclique aromatique ayant de 1 à 3 atomes de N, O et/ou S qui est mono- ou di-substitué par Hal,
R¹ désigne A, qui peut être mono-, di- ou trisubstitué par S(O)ₘA, Ph, NH₂, NHA, NA₂, OH, OA, PO(OA)₂, éthynyle, vinyle ou O(CH₂)ₙPh,
R² désigne H, Hal ou A,
R³ désigne 2-oxopipéridin-1-yle, 2-oxopyrrolidin-1-yle, 2-oxo-1*H-*pyridin-1-yle, 3-oxomorpholin-4-yle, 4-oxo-1*H*-pyridin-1-yle, 2-oxo-1*H*-pyrazin-1-yle, 2-oxoimidazolidin-1-yle, 2-iminopipéridin-1-yle, 2-iminopyrrolidin-1-yle, 3-iminomorpholin-4-yle, 2-iminoimidazolidin-1-yle, 2-imino-1*H*-pyrazin-1-yle, 2,6-dioxo-pipéridin-1-yle, 2-oxopipérazin-1-yle, 2,6-dioxopipérazin-1-yle, 2,5-dioxopyrrolidin-1-yle, 2-oxo-1,3-oxazolidin-3-yle, 3-oxo-2H-pyridazin-2-yle, 2-caprolactam-1-yle (= 2-oxoazépan-1-yl), 2-azabicyclo[2.2.2]octan-3-on-2-yle, 5,6-dihydro-1*H*-pyrimidin-2-oxo-1-yle, 2-oxo-1,3-oxazinan-3-yle ou 4*H*-1,4-oxazin-4-yle,
A désigne H, alkyle non ramifié, ramifié ou cyclique ayant 1-10 atomes de C,
Ph désigne phényle,
Hal désigne F, Cl, Br ou I,
n désigne 1, 2, 3, 4, 5 ou 6,
m désigne 0, 1 ou 2,
ainsi que les solvats, sels et stéréoisomères pharmaceutiquement utilisables de ceux-ci, y compris des mélanges de ceux-ci en toutes proportions.

2. Composés selon la revendication 1, dans lesquels
R¹ désigne alkyle ayant 1, 2, 3, 4, 5 ou 6 atomes de C, pouvant être substitué par éthynyle, phényle, OA, OH ou OA,
ainsi que les solvats, sels et stéréoisomères pharmaceutiquement utilisables de ceux-ci, y compris des mélanges de ceux-ci en toutes proportions.

3. Composés selon la revendication 1, dans lesquels
R³ désigne 2-oxopipéridin-1-yle, 2-oxopyrrolidin-1-yle, 2-oxo-1*H-*pyridin-1-yle, 3-oxomorpholin-4-yle, 4-oxo-1*H*-pyridin-1-yle, 2-oxo-1*H*-pyrazin-1-yle, 2-oxoimidazolidin-1-yle, 2-iminopipéridin-1-yle, 2-iminopyrrolidin-1-yle, 3-iminomorpholin-4-yle, 2-iminoimidazolidin-1-yle, 2-imino-1*H*-pyrazin-1-yle, 2-oxopipérazin-1-yle ou 3-oxo-2H-pyridazin-2-yle,
ainsi que les solvats, sels et stéréoisomères pharmaceutiquement utilisables de ceux-ci, y compris des mélanges de ceux-ci en toutes proportions.

4. Composés selon l'une ou plusieurs parmi les revendications 1-3, dans lesquels
R² désigne H, méthyle ou F,
ainsi que les solvats, sels et stéréoisomères pharmaceutiquement utilisables de ceux-ci, y compris des mélanges de ceux-ci en toutes proportions.

5. Composés selon l'une ou plusieurs parmi les revendications 1-4, dans lesquels
Hét désigne thiényle, furyle, pyrrolyle, benzofuranyle, benzo[b]thiényle, thiazolyle ou oxazolyle, chacun d'entre eux étant mono- ou disubstitué par Hal,
ainsi que les solvats, sels et stéréoisomères pharmaceutiquement utilisables de ceux-ci, y compris des mélanges de ceux-ci en toutes proportions.

6. Composés selon l'une ou plusieurs parmi les revendications 1-5, dans lesquels
Hét désigne thiényle, furyle, pyrrolyle, benzofuranyle, benzo[b]-thiényle, thiazolyle ou oxazolyle, chacun d'entre eux étant mono- ou disubstitué par Hal,
R¹ désigne alkyle ayant 1, 2, 3, 4, 5 ou 6 atomes de C, pouvant être substitué par éthynyle, phényle, OA, OH ou OA,
R² désigne H, Hal ou A,
R³ désigne 2-oxopipéridin-1-yle, 2-oxopyrrolidin-1-yle, 2-oxo-1*H-*pyridin-1-yle, 3-oxomorpholin-4-yle, 4-oxo-1*H*-pyridin-1-yle, 2-oxo-1*H*-pyrazin-1-yle, 2-oxoimidazolidin-1-yle, 2-iminopipéridin-1-yle, 2-iminopyrrolidin-1-yle, 3-iminomorpholin-4-yle, 2-iminoimidazolidin-1-yle, 2-imino-1*H*-pyrazin-1-yle, 2-oxopipérazin-1-yle ou 3-oxo-2H-pyridazin-2-yle,
A désigne H, alkyle non ramifié, ramifié ou cyclique ayant 1-10 atomes de C,
Ph désigne phényle,
Hal désigne F, Cl, Br ou I,
n désigne 1, 2, 3, 4, 5 ou 6,
m désigne 0, 1 ou 2,
ainsi que les solvats, sels et stéréoisomères pharmaceutiquement utilisables de ceux-ci, y compris des mélanges de ceux-ci en toutes proportions.

7. Composés selon la revendication 1, choisis parmi le groupe constitué par
le 1-(5-chlorothién-2-ylcarbonyl)-4-[4-(3-oxomorpholin-4-yl)phényl]-2-propylsemicarbazide,
le 1-(5-chlorothién-2-ylcarbonyl)-4-[3-méthyl-4-(3-oxomorpholin-4-yl)phényl]-2-(prop-2-ynyl)semicarbazide,
le 1-(3-chlorothién-2-ylcarbonyl)-4-[3-méthyl-4-(3-oxomorpholin-4-yl)phényl]-2-benzylsemicarbazide,
le 1-(5-bromothién-2-ylcarbonyl)-4-[3-méthyl-4-(3-oxomorpholin-4-yl)phényl]-2-benzylsemicarbazide,
le 1-(3-chlorobenzo[b]thiényl-2-ylcarbonyl)-4-[3-méthyl-4-(3-oxomorpholin-4-yl)phényl]-2-benzylsemicarbazide,
le 1-(5-chlorothién-2-ylcarbonyl)-4-[3-méthyl-4-(3-oxomorpholin-4-yl)phényl]-2-benzylsemicarbazide,
le 1-(5-bromothién-2-ylcarbonyl)-4-[3-fluoro-4-(3-oxomorpholin-4-yl)phényl]-2-(2-méthoxyéthyl)semicarbazide,
le 1-(3-chlorobenzo[b]thiényl-2-ylcarbonyl)-4-[3-fluoro-4-(3-oxomorpholin-4-yl)phényl]-2-(2-méthoxyéthyl)semicarbazide,
le 1-(3-chlorothién-2-ylcarbonyl)-4-[3-fluoro-4-(3-oxomorpholin-4-yl)phényl]-2-(2-méthoxyéthyl)semicarbazide,
le 1-(5-chlorothién-2-ylcarbonyl)-4-[3-fluoro-4-(3-oxomorpholin-4-yl)phényl]-2-(2-méthoxyéthyl)semicarbazide,
le 1-(3-chlorothién-2-ylcarbonyl)-4-[4-(2-oxo-1*H*-pyrazin-1-yl)-phényl]-2-cyclopropylméthylsemicarbazide,
le 1-(3-chlorothién-2-ylcarbonyl)-4-[4-(2-oxo-1*H*-pyridin-1-yl)-phényl]-2-cyclopropylméthylsemicarbazide,
le 1-(3-chlorothién-2-ylcarbonyl)-4-[3-méthyl-4-(3-oxomorpholin-4-yl)phényl]-2-cyclopropylméthylsemicarbazide,
le 1-(5-chlorothién-2-ylcarbonyl)-4-[3-méthyl-4-(3-oxomorpholin-4-yl)phényl]-2-(2-méthoxyéthyl)semicarbazide,
le 1-(5-chlorothién-2-ylcarbonyl)-4-[3-méthyl-4-(3-oxomorpholin-4-yl)phényl]-2-cyclopropylméthylsemicarbazide,
le 1-(5-bromothién-2-ylcarbonyl)-4-[3-méthyl-4-(3-oxomorpholin-4-yl)phényl]-2-cyclopropylméthylsemicarbazide,
ainsi que les solvats, sels et stéréoisomères pharmaceutiquement utilisables de ceux-ci, y compris des mélanges de ceux-ci en toutes proportions.

8. Procédé de préparation de composés de formule I selon les revendications 1-7 et des solvats, sels et stéréoisomères pharmaceutiquement utilisables de ceux-ci, **caractérisé en ce que**
a) un composé de formule II 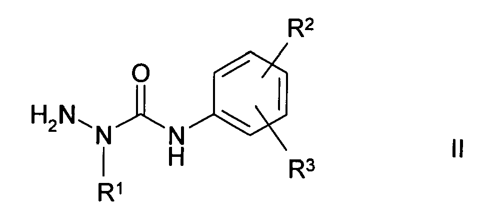
dans laquelle
R¹, R² et R³ ont la signification indiquée selon la revendication 1,
est réagi avec un composé de formule III
Hét-CO-L III
dans laquelle
L désigne Cl, Br, I ou un groupement OH libre ou modifié de manière réactive et fonctionnelle, et
Hét a la signification indiquée selon la revendication 1,
et/ou
une base ou un acide de formule I est converti(e) en l'un de ses sels.

9. Composés de formule I selon l'une ou plusieurs parmi les revendications 1 à 7, comme inhibiteurs du facteur de coagulation Xa.

10. Composés de formule I selon l'une ou plusieurs parmi les revendications 1 à 7, comme inhibiteurs du facteur de coagulation VIIa.

11. Médicaments comprenant au moins un composé de formule I selon l'une ou plusieurs parmi les revendications 1 à 7 et/ou des solvats, sels et stéréoisomères pharmaceutiquement utilisables de ceux-ci, y compris des mélanges de ceux-ci en toutes proportions, et, si on le souhaite, des excipients et/ou adjuvants.

12. Médicaments comprenant au moins un composé de formule I selon l'une ou plusieurs parmi les revendications 1 à 7 et/ou des solvats, sels et stéréoisomères pharmaceutiquement utilisables de ceux-ci, y compris des mélanges de ceux-ci en toutes proportions, et au moins un autre ingrédient actif médicamenteux.

13. Utilisation de composés selon les revendications 1 à 7 et/ou de sels et solvats physiologiquement acceptables de ceux-ci, pour la préparation d'un médicament destiné au traitement des thromboses, de l'infarctus du myocarde, de l'artériosclérose, des inflammations, de l'apoplexie, de l'angine de poitrine, de la resténose après angioplastie, de la boiterie intermittente, de la migraine, des tumeurs, des maladies tumorales et/ou des métastases tumorales.

14. Ensemble (kit) constitué de conditionnements séparés
(a) d'une quantité efficace d'un composé de formule I selon l'une ou plusieurs parmi les revendications 1 à 7 et/ou de solvats, sels et stéréoisomères pharmaceutiquement utilisables de ceux-ci, y compris des mélanges de ceux-ci en toutes proportions,
et
(b) d'une quantité efficace d'un autre ingrédient actif médicamenteux.

15. Utilisation de composés de formule I selon l'une ou plusieurs parmi les revendications 1 à 7 et/ou de solvats, sels et stéréoisomères pharmaceutiquement utilisables de ceux-ci, y compris des mélanges de ceux-ci en toutes proportions,
pour la préparation d'un médicament destiné au traitement des thromboses, de l'infarctus du myocarde, de l'artériosclérose, des inflammations, de l'apoplexie, de l'angine de poitrine, de la resténose après angioplastie, de la boiterie intermittente, de la migraine, des tumeurs, des maladies tumorales et/ou des métastases tumorales, en association avec au moins un autre ingrédient actif médicamenteux.
